Europäisches Patentamt

European Patent Office

Office européen des brevets

(11) Numéro de publication : **0 511 033 A1**

# (12) DEMANDE DE BREVET EUROPEEN

(21) Numéro de dépôt : **92400779.2**

(51) Int. Cl.$^5$ : **A61K 7/48, A61K 35/78**

(22) Date de dépôt : **23.03.92**

(30) Priorité : **03.04.91 FR 9104011**

(43) Date de publication de la demande :
**28.10.92 Bulletin 92/44**

(84) Etats contractants désignés :
**AT BE CH DE DK ES FR GB GR IT LI LU MC NL PT SE**

(71) Demandeur : **SYNTHELABO**
**22, Avenue Galilée**
**F-92350 Le Plessis Robinson (FR)**

(72) Inventeur : **Favre, Bernard**
**4, rue du Pressoir**
**F-37270 Saint-Martin-le-Beau (FR)**
Inventeur : **Potier, Anne**
**39-41, rue Emile Zola Appt 10**
**F-37100 Tours (FR)**
Inventeur : **Fontanel, Didier**
**16, rue de la Gatine**
**F-37390 Notre Dame d'Oe (FR)**
Inventeur : **Tanguy, Gilles**
**13, rue Caulaincourt**
**F-37100 Tours (FR)**

(74) Mandataire : **Thouret-Lemaitre, Elisabeth et al**
**SYNTHELABO Service Brevets 22, avenue Galilée**
**F-92352 LE PLESSIS ROBINSON CEDEX (FR)**

(54) **Extraits d'alchemille, leur préparation et leurs applications.**

(57)   Extrait d'Alchemille, caractérisé en ce qu'il contient de l'acide gallique et des tanins galliques, et par le fait que la teneur en acide gallique est de 0,2 à 1 % en poids, et la teneur en tanins galliques est de 6 à 15 % en poids, par rapport à la poudre pour un extrait sec et par rapport au résidu sec pour un extrait liquide ou mou.
   Application en cosmétique.

EP 0 511 033 A1

Jouve, 18, rue Saint-Denis, 75001 PARIS

La présente invention a pour objet l'obtention d'extraits d'Alchemille, *Alchemilla vulgaris L.,* les extraits ainsi obtenus et leurs applications.

L'Alchemille est une plante vivace de 10 à 40 cm de hauteur, très polymorphe. On la rencontre dans toute l'Europe à l'exception de la zone méditerranéenne, dans les régions tempérées, en Afrique australe, en Asie septentrionale, dans la partie orientale de l'Amérique du Sud, au Groënland et au Labrador.

La racine est assez grosse, dure, fibreuse, noirâtre et chevelue. Les fleurs sont petites, vert jaunâtre, disposées en bouquets corymbiformes au sommet des tiges et de leurs rameaux. Les feuilles sont glabres, arrondies et bordées de 7 à 11 lobes arrondis. Deux stipules dentées accompagnent chaque feuille.

La plante est récoltée à l'état sauvage, en Asie occidentale et en Europe orientale. La récolte s'effectue pendant toute la durée de la végétation.

Les parties de plante utilisées sont les feuilles et les tiges.

Les principaux constituants des feuilles et des tiges d'Alchemille sont :
- des acides phénoliques
    - acide salicylique
    - acide gallique,
- des acides gras
    - acides palmitique et stéarique,
- des stérols
    - phytostérol et tocophérol (vitamine E),
- des amino-acides,
- des tanins galliques.

Les extraits d'Alchemille de l'invention sont obtenus selon le procédé décrit ci-dessous :

Selon l'invention , on soumet les parties aériennes (feuilles et tiges) d'Alchemille fraîches ou sèches, broyées ou pulvérisées, à une extraction par un solvant tel que l'eau, un alcanol en $C_1$-$C_4$, l'acétone ou le propylèneglycol, ou un mélange de ces solvants. Par exemple, on peut utiliser un mélange alcanol $C_1$-$C_4$/eau ou un mélange acétone/eau en proportions de 96/4 à 30/70 en volume. On peut également utiliser un mélange propylèneglycol/eau dans des proportions de 90/10 à 40/60 en volume.

La quantité de solvant d'extraction utilisée est égale à 4 à 20 fois le poids des parties aériennes d'Alchemille. On effectue l'extraction éventuellement sous agitation à une température située entre 10°C et l'ébullition du solvant. La durée de l'extraction est de 2 à 80 heures. On concentre éventuellement les solutions extractives. On sèche éventuellement les concentrats obtenus, soit en étuve sous vide, soit par nébulisation, soit par lyophilisation, soit par séchage à l'aide d'un sécheur-réacteur cylindrique.

Les extraits des parties aériennes d'Alchemille obtenus selon l'invention sont caractérisés par la présence d'acides phénoliques tel que l'acide gallique, ainsi que par la présence de tanins galliques.

On caractérise ces constituants à l'aide des expériences suivantes :

On prépare une solution hydroéthanolique (à 60 % v/v d'éthanol) à 2% d'un extrait des parties aériennes d'Alchemille obtenu selon l'invention. L'addition de cyanure de potassium provoque l'apparition d'une coloration orangée, caractéristique de l'acide gallique. La même solution donne ensuite un précipité de couleur marron-jaune qui caractérise la présence de tanins. L'addition de chlorure ferrique provoque l'apparition d'un précipité bleu-noir caractéristique des tanins galliques.

On caractérise également l'acide gallique par chromatographie sur couche mince de gel de silice. On utilise une phase mobile constituée d'un mélange de n-butanol, d'acide acétique et d'eau, dans les proportions 4-1-5. On pulvérise sur la plaque chromatographique une solution hydroéthanolique (à 96 % v/v d'éthanol) à 2 % de chlorure ferrique, ce qui révèle la présence d'acide gallique.

On dose les tanins par spectrophotométrie après réaction à l'acide phototungstique avant et après adsorption sur poudre de peau. Cette technique de dosage est développée dans la Pharmacopée Française, Xème édition, pour le dosage des tanins dans les racines de ratanhia.

On dose les tanins par rapport à un témoin pur d'acide gallique. Les teneurs en tanins varient, selon la nature de l'extrait obtenu selon l'invention, de 6 à 15 % en poids par rapport à la poudre pour un extrait sec et par rapport au résidu sec pour un extrait liquide ou mou.

On dose l'acide gallique par chromatographie liquide haute performance. On utilise une colonne de type silice phase greffée $C_{18}$, et une phase mobile composée d'un mélange à proportions variables de méthanol, d'eau et d'acide acétique ; le débit est de 1 millitre par minute ; la détection s'effectue à 254 nm. Les teneurs en acide gallique varient de 0,2 à 1 % en poids par rapport à la poudre pour un extrait sec et par rapport au résidu sec pour un extrait liquide ou mou.

Les exemples suivants illustrent l'invention.

EXEMPLE 1

On introduit dans un réacteur chauffant 20 kg de feuilles et de tiges d'Alchemille, ainsi que 300 litres d'eau. On chauffe et on maintient une température de 80°C pendant 2 heures. On récupère les liqueurs d'extraction, on les filtre puis on les concentre sous vide à 60°C. On nébulise le concentrat. On obtient une poudre marron de saveur légèrement piquante. On identifie l'acide gallique par chromatographie sur couche mince. On identifie également les tanins et l'acide gallique par leur réaction colorée respective, puis on les dose suivant la méthode décrite précédemment. La teneur en acide gallique est 0,6 % en poids, celle des tanins est de 10 % en poids par rapport au produit sec.

EXEMPLE 2

On introduit dans un percolateur 10 kg de feuilles et de tiges d'Alchemille ainsi que 100 kg d'une solution hydroéthanolique à 30 % v/v d'éthanol. Après une macération de 12 heures, on procède à l'écoulement du solvant pendant 24 heures. On filtre le lixiviat puis on le concentre sous vide à 60°C jusqu'à l'obtention d'un résidu sec correspondant à 80% du filtrat. On identifie les tanins galliques et l'acide gallique par leur réaction colorée respective. On identifie également l'acide gallique par chromatographie sur couche mince, et on le dose suivant la méthode décrite précédemment. La teneur en acide gallique par rapport au produit sec est de 0,5 % en poids. On dose également les tanins galliques suivant la méthode décrite précédemment. Leur teneur est de 11 % en poids par rapport au produit sec.

Les extraits d'Alchemille obtenus selon l'invention présentent une activité filtre U.V. ainsi qu'une activité anti-radicalaire.

On démontre l'activité filtre U.V. des extraits obtenus selon l'invention à partir des observations suivantes :

Le spectre U.V. des extraits secs ou concentrés d'Alchemille solubilisés dans une solution aqueuse ou hydroalcoolique, ainsi que celui des extraits liquides, présente une zone d'absorption entre 220 et 400 nm avec 2 maxima d'absorption. Le premier situé entre 240 et 260 nm, le second entre 320 et 360 nm.

L'activité des extraits d'Alchemille selon l'invention est une activité filtre U.V. large, puisqu'elle couvre les zones des U.V.A et des U.V.B. Cette activité de type large est une particularité des extraits d'Alchemille selon l'invention.

Les extraits d'Alchemille obtenus selon l'invention présentent également des activités anti-radicalaires. On a démontré ces activités in vitro par deux méthodes de dosage. On a réalisé la première méthode de dosage sur un radical libre : le diphénylpicrylhydrazyl hydrate qui absorbe dans le violet à 513 nm. (Lamaison JL., Petitjean-Freytet C., Carnat P., Carnat A., 1988, Plantes Médicinales et Phytothérapie, 22, (4), p. 231-234).

Un composé anti-radicalaire, piégeur des radicaux libres, entraîne une inhibition d'apparition de coloration du radical libre témoin, donc une diminution de la densité optique de ce radical libre. L'activité anti-radicalaire d'une substance s'exprime par la diminution du pourcentage d'absorbance du radical libre testé. Dans ces conditions de test, un extrait d'Alchemille obtenu selon l'invention présente une activité similaire à la vitamine E qui est la substance de référence la plus utilisée. La cynarine, qui est la substance de référence la plus performante, présente une activité légèrement supérieure à la vitamine E et donc aux extraits d'Alchemille.

| SUBSTANCES TESTEES | CONCENTRATION mg/ml | INHIBITION % |
|---|---|---|
| | 0,01 | 9 |
| | 0,05 | 28 |
| Vitamine E | 0,1 | 61 |
| | 0,5 | 95 |
| | 1 | 96 |
| | 0,01 | 16 |
| | 0,05 | 60 |
| Cynarine | 0,1 | 89 |
| | 0,5 | 94 |
| | 1 | 96 |
| | 0,01 | 11 |
| Extrait aqueux d'Alchemille caractérisé en ce que la | 0,05 | 32 |
| teneur en acide gallique est de 0,6 % en poids et la te- | | |
| neur en tanins galliques est de 10 % en poids par rap- | 0,1 | 55 |
| port au produit sec. | 0,5 | 93 |
| | 1 | 95 |

On a également testé l'activité anti-radicalaire sur l'anion superoxyde généré par la réaction de NADH (nicotinamide adénine dinucléotide hydrate) sur le PMS (phénazine méthosulfate). L'anion superoxyde provoque la réduction du NBT (Nitrobleu de Tétrazolium) en diformazan, de coloration bleu intense.

On dose celui-ci par spectrophotométrie (Roback J., Gryglewski RJ., Biomedical Pharmacology, 1988, 37, (5), P. 837-841). On exprime l'activité anti-radicalaire d'une substance en pourcentage d'inhibition du développement de coloration due au diformazan. Par cette méthode de dosage, les extraits d'Alchemille présentent une activité bien supérieure aux 2 témoins testés, qui sont la quercétine et l'acide rosmarinique. Ainsi, on obtient 90 % d'inhibition avec des concentrations respectivement de 0,5 mg/ml d'acide rosmarinique et de quercétine, alors qu'on atteint ce niveau d'inhibition à une concentration de 0,1 mg/ml d'extrait aqueux d'Alchemille. Cet extrait est donc, dans ces conditions, 5 fois plus actif que les 2 témoins qui sont les plus actifs parmi les substances testées.

| SUBSTANCES TESTEES | CONCENTRATION mg/ml | INHIBITION % |
|---|---|---|
| Quercétine | 0,01 | 1 |
| | 0,05 | 31 |
| | 0,1 | 69 |
| | 0,5 | 91 |
| | 1 | 98 |
| Acide rosmarinique | 0,01 | 2 |
| | 0,05 | 28 |
| | 0,1 | 59 |
| | 0,5 | 90 |
| | 1 | 99 |
| Extrait aqueux d'Alchemille caractérisé en ce que la teneur en acide gallique est de 0,6 % en poids par rapport au produit sec, et la teneur en tanins galliques est de 10 % en poids par rapport au produit sec. | 0,01 | 35 |
| | 0,05 | 76 |
| | 0,1 | 92 |
| | 0,5 | 94 |
| | 1 | 96 |

La présence de tanins confèrent aux extraits d'Alchemille, en plus d'une activité cicatrisante, des propriétés antiprurigineuse, adoucissante et émolliente.

Ces propriétés sont complémentaires de l'activité filtre U.V.A. et U.V.B. et de l'activité anti-radicalaire.

Les extraits obtenus selon l'invention sont donc utilisables en cosmétique, en particulier pour la protection de la peau exposée à un ensoleillement prolongé et donc agressée par les U.V.A et les U.V.B. : l'activité anti-radicalaire des extraits d'Alchemille selon l'invention permet de lutter efficacement contre les effets néfastes des U.V.A, qui provoquent l'apparition de radicaux libres toxiques responsables d'un vieillissement prématuré de la peau. Les activités cicatrisante, anti-prurigineuse, adoucissante et émolliente des extraits selon l'invention permettent de soulager et de combattre l'inflammation des tissus cutanés due aux U.V.B. Ces activités renforcent l'effet filtre U.V.A. et U.V.B. des extraits d'Alchemille selon l'invention.

Les extraits de parties aériennes (feuilles et tiges) d'Alchemille de l'invention peuvent être utilisés sous forme de pommades ; préparées

- soit à partir d'un extrait d'Alchemille de l'invention, associé par exemple à un mélange d'excipients tels que monooléate de glycérol, vaseline, lanoline, cire blanche, huiles d'amande et de parrafine et eau,
- soit à partir d'un extrait d'Alchemille de l'invention, associé par exemple à un mélange d'excipients tels que huile d'arachide, lanoline anhydre, glycérine, amidon de blé, cire blanche et eau.

Les extraits d'Alchemille de l'invention peuvent aussi être présentés sous forme de crème dermique, lorsqu'ils sont associés à un mélange d'excipients tels que la cire d'abeille, la paraffine liquide légère, l'eau, la vaseline ou la lanoline, la cire blanche d'abeilles liquide légère, et/ou le glycérol.

Enfin, des formules de produits solaires peuvent être préparés à partir des extraits d'Alchemille de l'invention : on peut incorporer l'extrait dans des émulsions classiques H/E (huile/eau) ou E/H (eau/huile) ou préparer simplement des solutions huileuses d'extrait.

## Revendications

1. Extrait d'Alchemille, caractérisé par le fait qu'il contient de l'acide gallique et des tanins galliques, et par le fait que la teneur en acide gallique est de 0,2 à 1 % en poids, et la teneur en tanins galliques est de 6 à 15 % en poids, par rapport à la poudre pour un extrait sec et par rapport au résidu sec pour un extrait liquide ou mou.

2. Procédé d'obtention d'un extrait d'Alchemille selon la revendication 1, procédé caractérisé en ce que l'on extrait les parties aériennes (feuilles et tiges) d'Alchemille à l'aide d'un solvant tel que l'eau, l'acétone, un alcanol en $C_1$-$C_4$, le propylèneglycol ou un mélange de ces solvants.

3. Procédé selon la revendication 2, procédé caractérisé en ce que l'extraction est effectuée à l'aide d'un mélange alcanol en $C_1$-$C_4$/eau ou acétone/eau, mélange de 96/4 à 30/70 en volume.

4. Procédé selon la revendication 2, procédé caractérisé en ce que l'extraction est effectuée à l'aide d'un mélange propylèneglycol/eau, 100/10 à 40/60 en volume.

5. Procédé selon l'une quelconque des revendications 2 à 4, procédé caractérisé en ce que l'extraction est statique ou agitée.

6. Procédé selon l'une quelconque des revendications 2 à 5, procédé caractérisé en ce que la quantité de solvant utilisée égale 4 à 20 fois le poids des parties aériennes d'Alchemille.

7. Composition cosmétique, caractérisée en ce qu'elle contient un extrait d'Alchemille selon la revendication 1, en association avec tout excipient approprié.

8. Composition cosmétique selon la revendication 7, composition caractérisée en ce qu'elle présente une activité anti-radicalaire.

9. Composition cosmétique selon l'une quelconque des revendications 7 et 8, composition caractérisée en ce qu'elle présente une activité filtre UVA et UVB.

**EP 0 511 033 A1**

Office européen
des brevets

**RAPPORT DE RECHERCHE EUROPEENNE**

Numero de la demande

EP 92 40 0779

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | SEIFEN-ÖLE-FETTE-WACHSE, vol. 108, no. 15, septembre 1982, page A7, Augsburg, DE; "Cosmetochem" * Voir Frauenmantel * | 1-9 | A 61 K 7/48 A 61 K 35/78 |
| A | FR-A-2 332 026 (CARIEL) * En entier * | 1-9 | |
| A | GB-A-2 095 553 (INTERAG R.T.) * En entier * | 1-9 | |
| A | L. BEZANGER-BEAUQUESNE et al.: "Plantes médicinales des régions tempérées", 1980, page 197: "Alchemilla Vulgaris L.", Maloine S.A., Paris, FR * Paragraphe: "Principes actifs et emplois" * | 1-9 | |
| A | STN FILE SUPPLIER, KARLSRUHE DE, FICHIER CHEMICAL ABSTRACTS, vol. 102, no. 17, résumé no. 146198s, Columbus, Ohio, US; T. KRZACZEK: "Phenolic acids in some tannin drugs from the Rosaceae family", & FARM. POL., 1984, 40(8), 475-7 * Résumé * | 1-9 | DOMAINES TECHNIQUES RECHERCHES (Int. Cl.5) A 61 K |

Le présent rapport a été établi pour toutes les revendications

| Lieu de la recherche | Date d'achèvement de la recherche | Examinateur |
|---|---|---|
| LA HAYE | 02-07-1992 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

7